# EUROPEAN PATENT SPECIFICATION

(11) **EP 4 035 710 B1**
(45) Date of publication and mention of the grant of the patent: **31.12.2025**
(21) Application number: 19947116.0
(22) Date of filing: 25.09.2019
(51) Int. Cl.: A61M 5/20, A61M 5/145, A61M 5/50

(54) **SYRINGE PUMP, SYRINGE PUMP SYSTEM, SYRINGE PUMP CONTROL METHOD, AND STORAGE MEDIUM**
SPRITZENPUMPE, SPRITZENPUMPENSYSTEM, SPRITZENPUMPENSTEUERVERFAHREN UND SPEICHERMEDIUM
POMPE DE SERINGUE, SYSTÈME DE POMPE DE SERINGUE, PROCÉDÉ DE COMMANDE DE POMPE DE SERINGUE ET SUPPORT DE STOCKAGE

(43) Date of publication of application: 03.08.2022
(73) Proprietor: Shenzhen Mindray Scientific Co., Ltd., Shenzhen, Guangdong 518000 (CN)
(72) Inventor: JIANG, Xia, Shenzhen, Guangdong 518000 (CN); HE, Lijuan, Shenzhen, Guangdong 518000 (CN); PAN, Ruiling, Shenzhen, Guangdong 518000 (CN); MA, Lianbo, Shenzhen, Guangdong 518000 (CN); ZUO, Pengfei, Shenzhen, Guangdong 518000 (CN)
(74) Representative: KIPA AB
(86) International application number: PCT/CN2019/107847
(87) International publication number: WO 2021/056265

(56) References cited:
- EP-A1- 2 469 438
- WO-A1-2012/065649
- WO-A2-2013/176770
- CN-A- 101 644 567
- CN-A- 101 905 048
- CN-A- 102 940 914
- CN-A- 103 007 379
- CN-U- 204 072 959
- CN-Y- 2 566 870
- CN-Y- 201 008 675
- CN-Y- 201 094 786
- US-A1- 2002 019 612
- US-A1- 2016 296 699
- US-A1- 2019 096 518

## Description

### TECHNICAL FIELD

The disclosure relates to the technical field of medical devices, and more particularly to a syringe pump and a syringe pump system.

### BACKGROUND

Syringe pump is widely used because it can provide more accurate infusion tasks. The preparation work of the syringe pump before starting requires a long waiting time. It requires the user to start the syringe pump up, install the syringe and set the infusion parameters in turn. And the above operations are implemented in serial. Only when the previous operation satisfies the requirement, the next operation can be carried out. Finally, the infusion can be started. The overall preparation time is quite long. Especially in the case of emergency treatment for patients, such a long preparation time may even affect the treatment of patients.

EP 2469438 A1 provides a method to setup information to a user of a powered medical fluid injection system.

US 2002019612 A1 discloses an infusion pump having a function to prevent, when a door is opened and a midway part of a detachable infusion tube is to be temporarily held in a fixed position of a pump body, the door from being closed if the part is not properly set in the fixed position.

US 2019096518 A1 discloses a drug infusion device, which determines whether an infusion program is stored within a memory. If so, a user is prompted to confirm usage of the program and a drug identifier, and to review the configuration of the device. If the program is not in the memory, the drug infusion device is enabled to receive an auto program with which it can configure itself.

### SUMMARY

A syringe pump and a syringe pump system, which are capable of saving time for the infusion operation process, are provided in this disclosure.

The invention is defined by the subject-matter of claim 1. Further embodiments are defined in the dependent claims.

In the embodiments of this disclosure, the syringe pump controls the display screen to display the infusion parameter setting interface, when detecting that the pump door is closed and the installation state of the syringe inside the syringe pump is normal. During the operation process of the push-pull mechanism, the user can input and/or confirm the infusion parameters through the infusion parameter setting interface, which can save the preparation time before the syringe pump starts the infusion. In this way, it can also reduce the waste of the infusion time caused by waiting for the push-pull mechanism to run for a preset time to abut against the piston handle and then displaying the infusion parameter setting interface.

### BRIEF DESCRIPTION OF THE DRAWINGS

In order to explain embodiments of this disclosure more clearly, the following will briefly introduce drawings recited in the description for the embodiments or the prior art description. It is obvious that the drawings in the following description are only some embodiments of this disclosure. For those skilled in the art, other drawings can be obtained from these accompanying drawings without paying any creative works.
FIG. 1 is a step flowchart of a syringe pump control method in an embodiment not being part of the present disclosure.
FIG. 2 is a structural block diagram of a syringe pump in an embodiment of the present disclosure.
FIG. 3 is a hardware structure block diagram of a syringe pump in an embodiment of the present disclosure.
FIG. 4 is a structural diagram of a syringe pump in another embodiment of this disclosure.
FIG. 5 is a schematic diagram in which a syringe is installed inside a syringe pump in an embodiment of the present disclosure.
FIG. 6 is a schematic diagram showing a state in which a pump door of a syringe pump is closed in an embodiment of the present disclosure.
FIG. 7 is a schematic diagram showing a state in which a push-pull mechanism abuts against a piston handle in an embodiment of the present disclosure.
FIG. 8 is a schematic diagram when a syringe pump starts infusion operation in an embodiment of the present disclosure.

### DESCRIPTION OF THE PREFERRED EMBODIMENTS

The technical solutions in example embodiments of the disclosure will be described clearly and completely below with reference to the accompanying drawings. Apparently, the embodiments described are merely some, rather than all, of the embodiments of the disclosure. It should be understood that the disclosure is not limited by the example embodiments described herein. All other embodiments derived by those skilled in the art without creative efforts on the basis of the embodiments described in the disclosure shall fall within the scope of protection of the disclosure.

The expressions of "first" and "second" recited in the description, claims and accompanying drawings of this disclosure, are only to distinguish different objects, and do not intend to describe the specific sequence. In additional, the terms "comprising" and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these procedures, methods, or devices.

The expression "embodiment" when referred in the present disclosure, it means that the features, structures or characteristics described in connection with the embodiment may be included in at least one embodiment of the present disclosure. The phrase appearing in each position in this description does not necessarily refer to the same embodiment, nor refer to an independent or alternative embodiment mutually exclusive with other embodiments. Those skilled in the art explicitly and implicitly understand that the embodiments described herein can be combined with other embodiments. As used herein, depending on the context, the term "if" can be interpreted as meaning "when", "in response to determining" or "in response to detecting". Similarly, depending on the context, the phrase "if determined..." or "if [a stated condition or event] is detected" may be interpreted to mean "when determining...", "in response to determining...", "when [a stated condition or event] is detected" or "in response to a detection of [a stated condition or event]".

It should be noted that the following method embodiments not being part of the invention are expressed as a series of action combinations for simple description. However, those skilled in the art should be aware that the disclosure is not limited by the action sequence described herein, because according to the disclosure, some steps can be carried out in other sequences or at the same time.

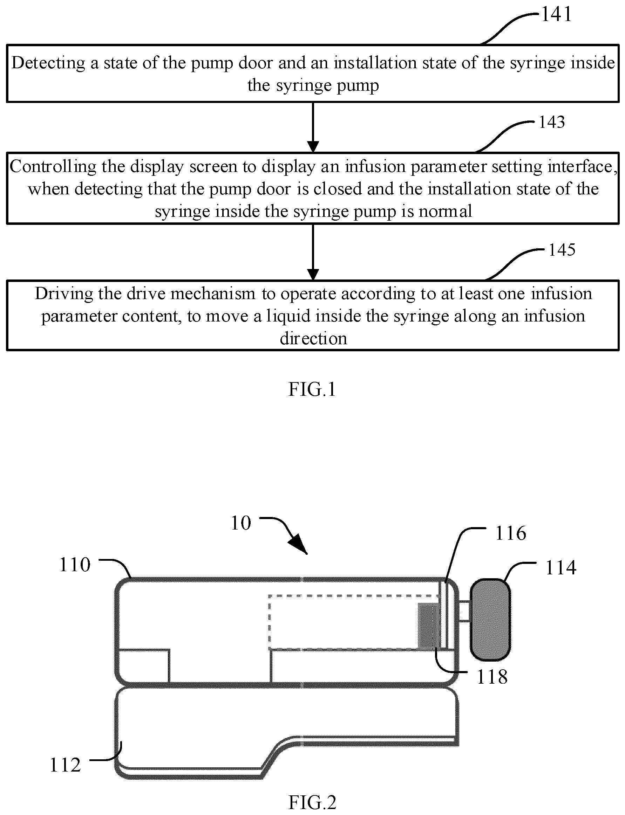

Referring FIG.1, a step flow diagram of a syringe pump control method in an embodiment not being part of the present disclosure is shown, which includes following steps.

In step S141, a state of the pump door and an installation state of the syringe inside the syringe pump are detected.

Now refer FIG.2 simultaneously, which is a hardware structure block diagram of a syringe pump in an embodiment of the present disclosure. The syringe pump 10 includes a pump body 110, a pump door 112 which is rotatably installed on the pump body 110, a handle clamping mechanism 118 which is installed inside the pump body 110, a receiving groove 116 which is installed at one side end of the pump body 110, and a push-pull mechanism 114 which is installed at the same side end of the pump body 110 as the receiving groove 116 and is movable along a preset direction (such as a left-right direction) under a control of a drive mechanism 133.

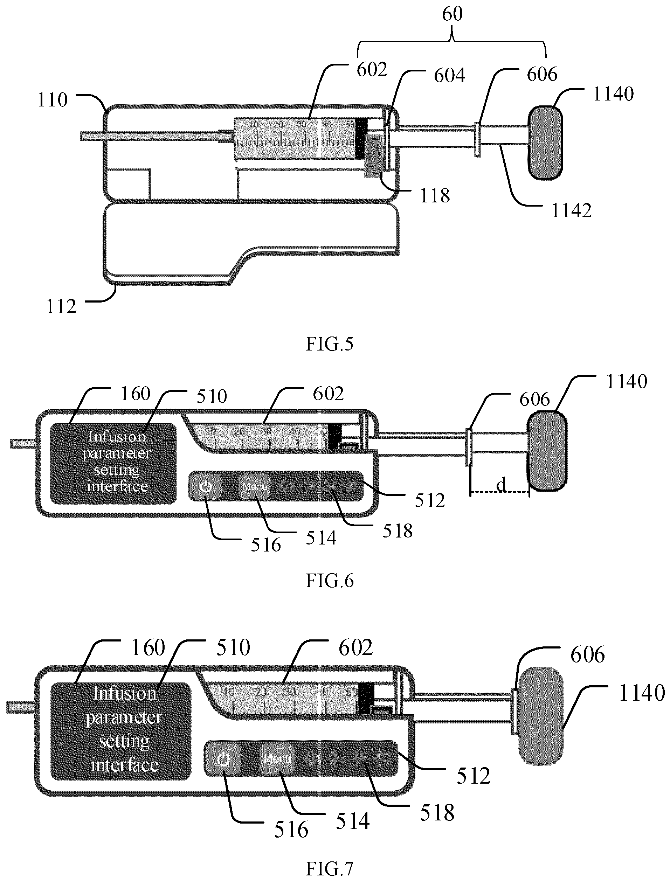

In this embodiment, the syringe pump 10 can work in a preparation phase of infusion operation or in a start-up phase of infusion operation. The preparation for infusion operation includes installing the syringe 60 (shown in FIG. 5) inside the syringe pump 10. After detecting that the installation state of the syringe 60 inside the syringe pump 10 is normal, the syringe pump 10 controls the push-pull mechanism 114 to move towards the piston handle 606 of the syringe 60. If the push-pull mechanism 114 contacts the piston handle 606 (shown in FIG. 5) of the syringe 60 and receives a start-up instruction for the infusion operation, the syringe pump 10 enters the start-up phase of the infusion operation. During the start-up phase of the infusion operation, the syringe pump 10 controls a liquid inside the syringe 60 to move in an infusion direction according to a preset infusion parameter setting.

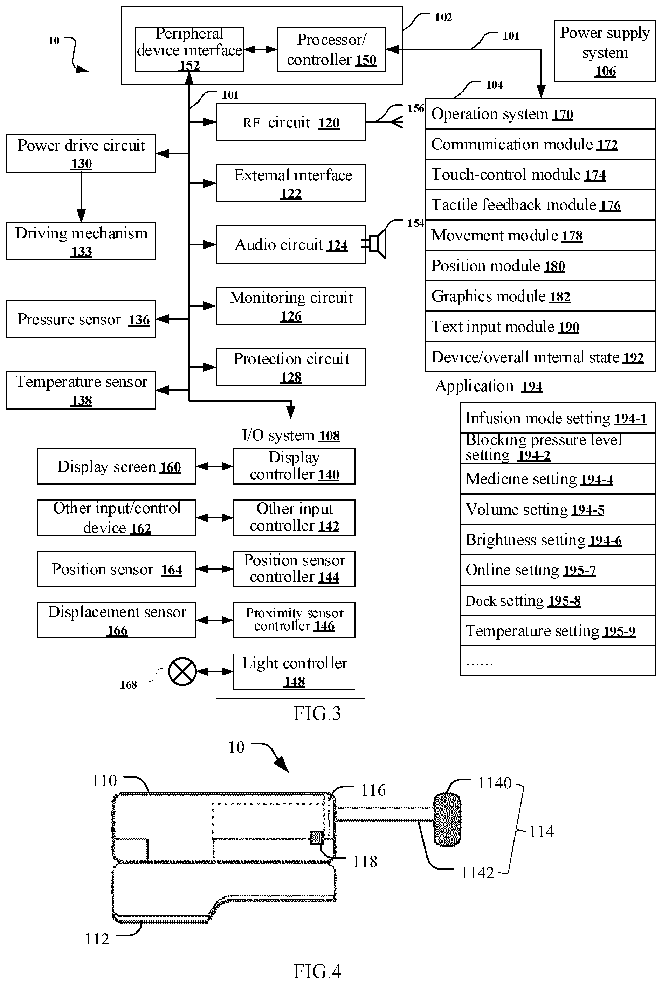

Now refer FIG.3 simultaneously, which is a hardware structure block diagram of a syringe pump in an embodiment of the present disclosure. The syringe pump 10 includes a control platform 102, a memory 104, a power supply system 106, an input/output (I/O) system 108, an RF circuit 120, an external interface 122, an audio circuit 124, a monitoring circuit 126, a protection circuit 128, a power drive circuit 130, a pressure sensor 136, a temperature sensor 138 and so on. These components communicate with each other through one or more communication buses or signal lines 101. The control platform 102 includes a processor/controller 150 and a peripheral device interface 152.

The syringe pump 10 may be any medical device that is capable of implementing infusion and injection operations which are preset by a user on a liquid which is prepared by the user, for infusing or injecting the prepared medicine liquid into a patient controllably. In some embodiments, the syringe pump 10 is used together with the syringe 60. It should be understood that the syringe pump 10 is only an example, and the syringe pump 10 may have more or fewer components than those are shown or have different component configurations. The various components described in FIG. 3 may be implemented in a hardware, a software, or a combination of software and hardware, and they can include one or more signal processing circuits and/or application specific integrated circuits.

The memory 102 may include a high-speed random-access memory and may also include a nonvolatile memory, such as one or more disk storage devices, flash memory devices, or other nonvolatile solid-state storage devices. In some embodiments, the memory 104 may also include a memory which is remote from one or more processers/controllers 150, such as an additional network memory accessed via the RF circuit 120 or via the external interface 122 and communication network (not shown). The communication network may be an Internet, one or more internal networks, a local area network (LAN), a wide area network (WLAN), a storage area network (SAN), etc., or an appropriate combination thereof. The processor/controller 150 may control access to the memory 104 by other components of the syringe pump 10 other than the peripheral device interface 152.

The peripheral device interface 152 couples input and output peripherals of the syringe pump 10 to the processor/controller 150 and the memory 104. For example, the peripheral device interface 152 may include an input interface and an output interface. The one or more processor/controllers 150 run various software programs and/or instruction groups stored in the memory 104 to perform various functions of the syringe pump 10 and process data.

In some embodiments, the peripheral device interface 152 and the processor/controller 150 may be implemented on a single chip. In some embodiments, they can be implemented on multiple discrete chips. The processor 150 can be a central processing unit (CPU), other general-purpose processors, digital signal processors (DSP), application specific integrated circuits (ASIC), field programmable gate arrays (FPGA), or other programmable logic devices, discrete gate or transistor logic devices, discrete hardware components, etc. The general-purpose processor can be a microprocessor or the processor can also be any conventional processor, etc.

The RF (radio frequency) circuit 120 receives and transmits electromagnetic waves. The RF circuit 120 converts electrical signals into electromagnetic waves, or converts electromagnetic waves into electrical signals, and communicates with communication networks and other communication devices via electromagnetic waves. The RF circuit 120 may include well-known circuits for performing these functions, and these well-known circuits include but are not limited to the antenna system 156, a RF transceiver, one or more amplifiers, a tuner, one or more oscillators, a digital signal processor, a CODEC chipset, a user identity module (SIM) card, a memory, etc. The RF circuit 120 may communicate with networks and other devices through a wireless communication, which may be the world wide web (WWW), an intranet and/or a wireless network such as a cellular telephone network, a wireless local area network (LAN) and/or a metropolitan area network (MAN). The wireless communication can use any of a variety of communication standards, protocols and technologies, which include but are not limited to the global system for mobile communications (GSM), enhanced data GSM environment (EDGE), wideband code division multiple access (WCDMA), code division multiple access (CDMA), time division multiple access (TDMA), Bluetooth (such as ieee802.15.1), wireless fidelity (WiFi) (e.g., IEEE802.11a, IEEE 802.11b, IEEE 802.11g and/or IEEE 802.11n), voice over internet protocol (VoIP), WI-MAX, protocols for e-mail, instant messaging and/or short message service (SMS), or any other suitable communication protocols which include those are not yet developed at the date of submission of this disclosure.

The external interface 122 provides a wired communication interface between the syringe pump 10, other devices (such as a Dock, central station, monitor, etc.) or users (computers or other communication devices). In some embodiments, it can be a communication interface controlled by a CAN bus protocol, a communication interface controlled by a serial communication protocol (such as RS485, RS232), or a universal serial bus (USB). The external interface 122 is suitable for being directly or indirectly coupled to other devices or users via a network (such as the Internet, LAN, etc.).

The audio circuit 124 and the speaker 154 provide an audio interface between the user and the syringe pump 10. The audio circuit 124 receives audio data outputted from the peripheral device interface 152 through the output interface, converts the audio data into an electrical signal, and transmits the electrical signal to the speaker 154. The speaker 154 converts the electrical signal into a sound wave that can be perceived by human.

The monitoring circuit 126 may include a fault detection circuit which is operable to instruct a state of one or more processors/controllers 150.

The protection circuit 128 may include a hardware protection device (e.g., fuses, TVS diodes) which is operable to protect the electrical safety of various components inside the syringe pump 10. The processor/controller 150 drives the power device (such as the drive mechanism 133) of the syringe pump 10 through the power drive circuit 130, so that the power device can be moved controllably under the drive of the processor/controller 150. In the process of movement, one or more force transmission/conversion devices (such as gears or transmission shafts) drive the controlled object (such as a pump door, a push-pull mechanism 114) to move. The power device can be an electromagnetic device that realizes the electric energy conversion or transmission according to the law of electromagnetic induction, such as a permanent magnet (PM) motor, a reactive (VR) motor and a hybrid (HB) motor. In some embodiments, the motor is driven by the processor/controller 150 to enable the controlled object of the syringe pump 10 (such as the push-pull mechanism 114) to move, such that a preset movement state of the controlled object can be realized.

In some embodiments, the pressure sensor 136 may respond to a pressure value on the measured object (such as the pipe wall of the syringe 60), convert the pressure value into an electrical signal which is detectable and send it to the control platform 102. The pressure sensor 136 may be a resistance strain gauge pressure sensor, a semiconductor strain gauge pressure sensor, a piezoresistive pressure sensor, an inductive pressure sensor, a capacitive pressure sensor, a resonant pressure sensor, an optical fiber pressure sensor, or a capacitive acceleration sensor.

In some embodiments, the syringe pump 10 has a heating device for heating the liquid inside the syringe 60. At this time, the temperature sensor 138 can be operable to detect the real-time temperature of the liquid. At the same time, the temperature value is converted into an electrical signal which is detectable and then sent to the control platform 102. The control platform 102 can display the real-time temperature through the display screen 160 or control an on/off state of the heating device according to the temperature value.

The input/output (I/O) system 108 provides an interface between the input/output peripherals of the syringe pump 10 and the peripheral device interface 152. The input/output peripherals may be a display screen 160, a position sensor 164, a displacement sensor 166, a light component 168, and other input/control device 162. The I/O system 108 may include a display controller 140, a position sensor controller 144, a proximity sensor controller 146, a light controller 148, and one or more other input controllers 142. One or more controllers in the I/O system 108 receive/transmit electrical signals from/to input/output peripherals. One or more input controllers 142 receive/transmit electrical signals from/to other input/control devices 162. The other input/control devices 162 may include a physical key (such as, a press key, a rocker key or a touch key, etc.), a slider switch, a joystick, etc. In some embodiments, the other input/control device 162 may include a physical key for an emergency stop of the infusion.

In some embodiments, the display screen 160 provides an output interface between the syringe pump 10 and the user, and displays electronic documents on the screen through a specific transmission device and then reflects them to the human eye. The display screen can include a cathode ray pipe display (CRT), a plasma display (PDP) or a liquid crystal display (LCD), etc. In some embodiments, the display screen 160 may include a touch screen that provides an input/output interface between the syringe pump 10 and the user. The touch screen can be an inductive display device that can receive input signals such as contacts, and include a resistance screen, a surface acoustic wave screen, an infrared touch screen, an optical touch screen, a capacitive screen or a nano film. Both of the display screen and touch screen can display the visual output to the user, such as through the output interface in the peripheral device interface 152. Visual output optionally includes graphics, text, chart, video, and combinations thereof. Some or all visual outputs can correspond to user interface objects, which will be described in more detail in this disclosure.

The touch screen also receives an input of a user based on touch and/or contact. The touch screen forms a touch sensitive surface that receives the input of user. The touch screen and display controller 140 (together with any associated modules and/or instruction group in the memory 104) detects contact on the touch screen (and any movement or interruption of the touch) and converts the detected contact into an interaction with user interface objects such as one or more software keys displayed on the touch screen. In one exemplary embodiment, the contact point between the touch screen and the user corresponds to one or more fingers of the user. The touch screen can use LCD (liquid crystal display) technology or LPD (light emitting polymer display) technology. However, other display technologies can be used in other embodiments. The touch screen and display controller 140 may use any of a variety of touch sensitive technologies to detect the contact and its movement or interruption, which include but are not limited to capacitance, resistance, infrared and surface acoustic wave technologies, as well as other proximity sensor arrays, or other technologies for determining one or more points of contact with the touch screen.

The position sensor 164 can sense a position of the detected object, convert the position into an electrical signal which is detectable, and send the electrical signal to the control platform 102 through the I/O system 108. The position sensor 164 can be a contact sensor that generates a signal through contact and compression of two objects, such as a travel switch and a two-dimensional matrix position sensor; or can be a proximity sensor that generates a signal when two objects approach in a preset distance, such as an electromagnetic sensor, a photoelectric sensor, a differential transformer sensor, an eddy current sensor, a capacitive sensor, a reed switch sensor, an ultrasonic sensor or a hall sensor. The measured object can include the pump door, etc. In some embodiments, a hall position sensor may be operable to detect the position of the pump door.

The displacement sensor 166 can respond to a change of position of the measured object relative to a reference position, convert the change of position into an electrical signal which is detectable, and send the electrical signal to the control platform 102 through the I/O system 108. The displacement sensor 106 may be an inductive sensor, a capacitive sensor, an ultrasonic sensor or a hall sensor.

The light component 168 may include a visual alarm element which indicates that the syringe pump 10 is in an abnormal state. Light component 168 individually responds to the drive of the processor/controller 150. The light component 168 may also cooperate with the speaker 154 to respond to the drive of the processor/controller 150. For example, the color or brightness of the light changes with the tone and frequency of the alarm sound. The light component 168 may include indicators for a power supply, a CPU and other components or an alarm indicator which indicates the infusion failure state. The light component 168 may also include a visual lighting element for facilitating the observation of the structure or component state of the syringe pump 10 when the ambient light is poor.

The syringe pump 10 also includes a power supply system 106 for supplying power to various components. The power system 106 may include a power management system, one or more power sources (e.g., batteries or alternating current (AC)), a charging system, a power failure detection circuit, a power converter or inverter, a power state indicator (e.g., light emitting diodes (LED)), or any other component associated with power generation, management and distribution.

In some embodiments, the software components include an operation system 170, a communication module (or instruction group) 172, a touch-control module (or instruction group) 174, a tactile feedback module (or instruction group) 176, a movement module (or instruction group) 178, a position module (or instruction group) 180, a graphics module (or instruction group) 182, a text input module (or instruction group) 190, a device/overall internal state (or instruction group) 192, and one or more applications (instruction group) 194.

The operation system 170 (e.g., embedded operation systems, such as Darwin, RTXC, LINUX, UNIX, OS, WINDOWS, etc.) includes various software components and/or drivers for controlling and managing conventional system tasks (e.g., memory management, storage device control, power management, etc.) and facilitating communication between various software and hardware components.

The communication module 172 facilitates to communicate with other devices via one or more external interfaces 122, and it also includes various software components which are operable to process data received by the RF circuit 120 and/or the external interface 122.

In some embodiments, the touch-control module 174 may selectively detect contact with the display screen 160 or other touch sensitive devices (e.g., touch keys, touch pads). For example, the touch-control module 174 detects contact with the display screen 160 together with the display controller 140. The touch-control module 174 includes various software components which are operable to perform various operations associated with contact detection of the display screen 160 (which can be detected by a finger or a stylus, etc.), such as operations of determining whether the contact occurs (e.g., detecting a press time of a finger), determining a strength of the contact (e.g., a force or pressure of the contact), determining whether the contact moves (e.g., detecting one or more finger dragging events), tracking the movement on the display screen and determining whether the contact stops (e.g., detecting a lift time of the finger or a disconnection of the contact). Wherein the operation of determining a movement of a contact point may include determining a rate (amplitude), a speed (amplitude and direction), and/or an acceleration (including amplitude and/or direction) of the contact point. These operations can be applied to single-point contact or multi-point simultaneous contact. In some embodiments, the touch-control module 174 detects contact with other touch devices in conjunction with the display controller 140.

The touch-control module 174 may be operable to detect gesture inputted by the user. Different gestures of the user on the touch sensitive device have different contact modes (for example, a detected contact position, contact time or contact intensity, or combinations of one or more thereof). For example, detecting gesture of tapping by a single finger includes detecting a finger press event, and then detecting a finger lift event at the same or similar position as the finger press event. For example, detecting gesture of swiping by a finger includes detecting a finger press event, and then monitoring one or more finger dragging events, and finally detecting a finger lift event. Similarly, the gestures of the touch pen, such as tapping, swiping, dragging and so on, are optionally detected by detecting a designated contact graphics of the touch pen.

The tactile feedback module 176 includes various software components which are operable to generate instructions for generating a tactile output at one or more positions of the syringe pump 10 by using one or more tactile output generators (not shown), in response to the interaction of the user with the syringe pump 10. For example, after detecting the contact on the surface of the touch device, the color of the graphics or text of the touch device changes, or a sound or vibration generates.

The position module 180 includes software components which are operable to perform various operations which are related to the detection of the device position and the detection of the change of the device position.

The graphics module 182 includes various known software components which are operable to render or display a graphics on the display screen of the display screen 160 or other external devices, and includes components which are operable to change the visual impact (e.g., brightness, transparency, saturation, contrast or other visual attributes) of the displayed graphics. In the embodiments herein, the term "graphics" include any object that can be displayed to the user, which includes but is not limited to text, web page, icon (e.g., user interface objects of soft keys), digital image, video, animation, and the likes. In some embodiments, the graphics module 182 stores data which represents the graphics to be used. Each graphics can be assigned a corresponding code. The graphics module 182 receives one or more codes for the graphics which is designated to be displayed from an application or the like, and also receives coordinate data together with other graphics attribute data if necessary, and then generates screen image data for outputting to the display controller 140.

The text input module 190 provides various software components which are operable to input text into one or more applications. Specifically, it can be operable to input various infusion parameters, which include a medicine name, infusion speed or alarm threshold.

In some embodiments, the memory 104 stores the device/overall internal state 192. The device/overall internal state 192 includes one or more of the followings: an active application state that indicates which applications (if any) are currently active; a display state that indicates what applications, views, or other information occupy various areas of the display screen 160; a sensor state that includes information obtained from each sensor of the device and other input or control syringe pump 10; and position and/or orientation information about a position and/or posture of the device.

In some embodiments, the memory 104 stores at least one application 194, which may include an infusion mode setting 194-1, a blocking pressure level setting 194-2, a medicine setting 194-4, a volume setting 194-5, a brightness setting 194-6, an online setting 195-7, a Dock setting 195-8, or a temperature setting 195-9. The infusion mode setting 194-1 can include a combination of preset infusion parameters for satisfying requirements of different use scenarios. The blocking pressure level setting 194-2 can include an interface for the user to input different blocking pressure levels. By inputting different blocking pressures, the blocking alarm threshold of the syringe pump 10 can be adjusted for satisfying requirements of different use scenarios. The medicine setting 194-4 can include an interface for the user to input different medicine names, medicine abbreviations and/or medicine colors. The medicine parameters which are set before the infusion by inputting the corresponding medicine names/abbreviations/colors, can facilitate the automatic confirmation inside the syringe pump 10 or the verification of medical staff during infusion. The volume setting 194-5 allows the user to adjust the volume of alarm and/or other audio output according to their requirements. The brightness setting 194-6 allows the user to adjust the brightness of the screen, alarm indicator, lighting lamp and so on, according to their requirements. The online setting 195-7 provides an input interface for the user to control the syringe pump 10 to work online with other devices or not and to control an online working mode according to their requirements. The Dock setting 195-8 provides a setting interface for the user to adjust the working parameters of the Dock connected with the syringe pump 10 according to their requirements. The temperature setting 195-9 provides a setting interface for the user to heat the liquid in the infusion apparatus.

Please also refer to FIG. 4, which shows a structural diagram of a syringe pump in another embodiment of this disclosure. The push-pull mechanism 114 includes a connection rod 1142 and a push block 1140 which is fixed at one end of the connection rod 1142. The connection rod 1142 is slidably connected with the pump body 110 along its own axis. The push-pull mechanism 114 can move along an axial direction of the connection rod 1142 under the drive of the drive mechanism 133. The handle clamping mechanism 118 can be rotatably installed on the pump body 112. Under the rotation operation of the user, the handle clamping mechanism 118 can switch between a first state and a second state. Before installing the syringe 60, the user can rotate the handle clamping mechanism 118 from the first state (as shown in FIG. 2) to the second state (as shown in FIG. 4), and pull the push-pull mechanism 114 out for a preset length in a direction away from the pump body 110 (for example, after pulling the push-pull mechanism 114 in FIG.2 out for the preset length to the right, the push-pull mechanism 114 is located in the position shown in FIG. 4), So that the syringe pump 10 has enough space to install the syringe 60.

Please also refer to FIG. 5, which shows a schematic diagram of a syringe which is installed inside the syringe pump in an embodiment of this disclosure. In this embodiment, the syringe 60 includes a syringe body 602, a flange 604 which is installed at one end of the syringe body 602 and a piston handle 606. The syringe body 602 is hollow for receiving the piston handle 606, and the piston handle 606 can move in a radial direction of the syringe body 602. When installing the syringe 60 inside the syringe pump 10, the user can rotate the handle clamping mechanism 118 to the second state to press the syringe body 602 on the pump body 110 of the syringe pump. The flange 604 of the syringe 50 is received inside the receiving groove 116 of the pump body 110, and a distance between the piston handle 606 and the push block 1140 is a preset distance d (shown in FIG. 6).

In this embodiment, a position sensor 164 can be installed in the pump door 112, and the processor 150 determines a state of the pump door 112 through a first position sensor installed in the pump door 112, wherein the state of the pump door 112 includes an open state and a closed state. As shown in FIG. 2, the processor 150 can determine that the pump door 112 is opened by installing the first position sensor. Please also refer to FIG. 6, which is a state schematic diagram in which the pump door of the syringe pump is closed in an embodiment of this disclosure. As shown in FIG. 6, the processor 150 may determine that the pump door 112 is closed through the first position sensor.

In this embodiment, a second position sensor is installed inside the handle clamping mechanism 118. When the syringe 60, which is installed inside the syringe pump 10, is pressed on the pump body 110 of the syringe pump 10, the handle clamping mechanism 118 is in the second state. At this time, the second position sensor can transmit a feedback signal. Therefore, the processor 150 can detect whether the syringe 60 is installed inside the syringe pump 10 through the second position sensor. For example, when the syringe 60 is installed inside the syringe pump 10, the handle clamping mechanism 118 fixes the syringe body 602 on the pump body 110, and the second position sensor can output a feedback signal. In this way, the processor 150 can determine that the syringe body 602 of the syringe 60 is pressed on the pump body 110 of the syringe pump 10. In other embodiments, a pressure sensor can be installed in the handle clamping mechanism 118, that is, when the syringe 60 is pressed on the pump body 110 of the syringe pump 10, the pressure sensor can contact the syringe 60 to generate a feedback signal with pressure information. The processor 150 may also determine whether to press the syringe 60, which is installed inside the syringe pump 10, on the pump body 110 of the syringe pump 10 according to the received feedback signal with pressure information.

In this embodiment, a third position sensor is installed in the receiving groove 116. When the syringe 60 is installed inside the syringe pump 10, the flange 604 of the syringe 60 is received inside the receiving groove 116. At this time, the third position sensor can also output a feedback signal. Thus, when the processor 150 receives the feedback signal outputted by the third position sensor, the processor 150 can determine that the flange 604 of the syringe 60 is received inside the receiving groove 116.

Further, the processor 150 can detect the state of the pump door 112 according to the first position sensor, and can also detect an installation state of the syringe 60 according to the second position sensor and the third position sensor. In this embodiment, when both the second position sensor and the third position sensor transmit feedback signals to the processor 150, the processor 150 can determine that the installation state of the syringe 60 is normal. When the processor 150 does not receive the feedback signal transmitted by either of the second position sensor and the third position sensor, the processor 150 can determine that the installation state of the syringe 60 is abnormal. For example, when the processor 150 receives the feedback signal transmitted by the second position sensor but does not receive the feedback signal transmitted by the third position sensor, it indicates that the flange 604 of the syringe 60 is received inside the receiving groove 116, and the installation state of the syringe 60 is abnormal. When the processor 150 receives the feedback signal transmitted by the third position sensor but does not receive the feedback signal transmitted by the second position sensor, it indicates that the user does not switch the handle clamping mechanism 118 to the second state, and the handle clamping mechanism 118 does not fix the syringe body 602 on the pump body 110. Further, the processor 150 can also detect that the installation state of the syringe 60 is abnormal. When determining that the installation state of the syringe 60 is abnormal, the processor 150 controls the display screen 160 to output reminding information of the abnormal installation state of the syringe 60. The processor 150 can also control the light assembly 168 to output the reminding information of the abnormal installation state of the syringe 60 through the light controller 148.

In one embodiment, if the pump door 112 is closed and the syringe 60 is not installed inside the syringe pump 10, the syringe pump 10 can be in a shutdown state or a standby state. If the user uses the syringe pump 10 to perform an infusion task of the syringe 60, the user needs to open the pump door 112 of the syringe pump 10. When the processor 150 detects that the pump door 112 is opened through the first position sensor, the processor 150 controls the syringe pump 10 to power on or start up, so that the syringe pump 10 is in a start-up state. During the period when the user installs the syringe 60 (i.e., the period from the time when the processor 150 detects that the pump door 112 is opened to the time when the processor 150 detects that the pump door 112 is closed), the syringe pump 10 can perform a self-inspection operation to detect a device state of the syringe pump 10. The self-inspection operation of the syringe pump 10 includes but is not limited to, sound self-inspection, processor self-inspection, external storage device self-inspection, alarm lamp self-inspection, power supply self-inspection and sensor self-inspection. During the sound self-inspection, the processor 150 can control the speaker 154 to make a sound through the audio circuit 124, and can determine whether the sound self-inspection passes through a recording device. If the processor receives the sound from the speaker 154 through the recording device, it can be determined that the sound self-inspection has passed. Otherwise, it indicates that the device state of the syringe pump 10 is abnormal. During the processor self-inspection, the processor 150 can run a preset program stored inside the memory 104 and determine whether the result obtained after running the preset program is a preset result. If the result obtained is the preset result, it can be determined that the processor self-inspection has passed. Otherwise, it indicates that the device state of syringe pump 10 is abnormal. In addition, the processor 150 can also detect the external storage device, the light assembly 168, the power supply system 106 and a plurality of sensors. When each self-inspection operation passes, it can be determined that the device state of the syringe pump 10 is normal. When at least one self-inspection operation fails, it is determined that the device state of the syringe pump 10 is abnormal, and the reminding information for the abnormal device state can be outputted through the display screen 160 or the light assembly 168.

Since the existing technical scheme may perform the self-inspection operation before installing the syringe 60 inside the syringe pump 10, that is, the pump door 112 is opened to install the syringe 60 inside the syringe pump 10, after the syringe pump 10 completes the self-inspection operation, which increases the time cost for the preparation phase of the infusion task (or the preparation phase of the infusion operation) to a certain extent. In this embodiment, when the pump door 112 is opened, the syringe pump 10 starts up, and the self-inspection operation is performed simultaneously during the installation of the syringe 60 inside the syringe pump 10, that is, the self-inspection operation of the syringe pump 10 is processed in parallel with the installation of the syringe 60, which is conducive to reducing the time cost for the preparation phase of the infusion task.

In other embodiments, the syringe pump 10 may include a start-up key which includes but is not limited to a physical keys or other types of keys). When the start-up key is triggered, the user can open the pump door 112 to install the syringe 60 inside the syringe pump 10. Then, the syringe pump 10 also performs a self-inspection operation while installing the syringe 60 inside the syringe pump 10, which can also reduce the time cost for the infusion task.

In step S143, the display screen is controlled to display an infusion parameter setting interface, when detecting that the pump door is closed and the installation state of the syringe inside the syringe pump is normal.

In this embodiment, when the processor 150 detects that the pump door 112 is closed and the installation state of the syringe 60 inside the syringe pump 10 is normal, the processor 150 can control the display screen 160 which is installed on the pump door 112 to display the infusion parameter setting interface 510, wherein the infusion parameter setting interface 510 includes an infusion parameter setting item which is operable to present one or more infusion parameter contents. Theses infusion parameter contents include but are not limited to an infusion speed, an infusion volume and other infusion parameters. When the pump door 112 is closed and the installation state of the syringe 60 inside the syringe pump 10 is normal, there is a preset distance d between the push block 1140 of the push-pull mechanism 114 and the piston handle 606. Therefore, when the drive mechanism 133 controls the push-pull mechanism 114 to start up the infusion operation, the drive mechanism 133 needs to move the push block 1140 towards the piston handle 606 for the preset distance d. After the push block 1140 abuts against (or contacts with) the piston handle 606, the processor 150 performs the infusion action (or infusion operation) according to a preset infusion parameter content. The existing technical scheme may control the display screen 160 to display the infusion parameter setting interface 510 after the push block 1140 abuts against the piston handle 606, so it takes a certain time for the push block 1140 to move for a preset distance to abut against the piston handle 606, which will also increase the time cost in the preparation phase of the infusion.

Please also refer to FIG. 7, which is a schematic diagram showing a state in which a push-pull mechanism abuts against a piston handle in an embodiment of the present disclosure. In this embodiment, when the installation state of the syringe 60 inside the syringe pump 10 is normal and the display screen 160 is controlled to display the infusion parameter setting interface 510 at the same time, the processor 150 drives the drive mechanism 133 to operate at a first motor speed, to enable the propulsion mechanism 114 to move towards the piston handle 606 of the syringe 60. After the drive mechanism 133 operates at the first motor speed for a preset time, the propulsion mechanism 114 abuts against the piston handle 606. In this way, the user controls the movement of the propulsion mechanism 114 while implementing the infusion parameter setting through the infusion parameter setting interface 510, which is also conducive to reducing the time cost for infusion operation.

In step 145, the drive mechanism is driven to operate according to at least one infusion parameter content, to move a liquid inside the syringe along an infusion direction.

In this embodiment, when the propulsion mechanism 114 abuts against the piston handle 606, the syringe pump 10 can enter the start-up phase of the infusion operation, and the processor 150 can drive the drive mechanism 133 to operate at a second motor speed according to at least one infusion parameter content, so as to control a relative movement between the piston handle 606 of the syringe 60 and the syringe body 602, so as to move the liquid inside the syringe 60 in the infusion direction.

In this embodiment, a plurality of display screens 160 can be installed on the pump door 112. For example, a first display screen and a second display screen are installed, in which the first display screen is operable to display the infusion parameter setting interface 510 and the second display screen is operable to display a function operation interface 512. As shown in FIG. 7, the function operation interface 512 includes a progress bar display area 518, a menu key area 514 and a switch key area 516. When the drive mechanism 133 drives the push-pull mechanism 114 for implementing the infusion operation, it indicates that the syringe pump 10 is implementing the infusion operation. At this time, the processor 150 can control the progress bar display area 518 to present first color information (such as green). When the infusion operation is completed, the processor 150 can control the progress bar display area 518 to present second color information (such as red). When the syringe pump 10 does not perform the infusion operation and the syringe 60 is not installed inside the syringe pump 10, the processor 150 can control the progress bar display area 518 to present third color information (such as gray).

The user can set or operate the contents in the infusion parameter setting interface 510 by triggering the menu key area 514, and can determine whether to start up the infusion operation. For example, when the user sets the infusion parameter setting interface 510, the processor 150 is operable to detect a trigger event of the user on the infusion parameter setting interface 510 and adjust the infusion parameter content based on the trigger event. When the drive mechanism 133 drives the push-pull mechanism 114 to move for a preset distance d at the first motor speed and then contact the piston handle 606, if the user is still adjusting the infusion parameter content, the drive mechanism 133 can pause driving the push-pull mechanism 114 at this time. After the user has completed the setting operation and send out a start-up instruction for the infusion, the processor 150 drives the drive mechanism 133 to operate according to the infusion parameter content, to start up the infusion operation. In this embodiment, the start-up instruction for the infusion can be generated when the user triggers a start-up key after setting the infusion parameter content, or can be sent by the Dock. For example, a plurality of syringe pumps can be connected in the Dock, and operable to perform the injection operations of a plurality of medicines in sequence. Dock can send a start-up instruction for the infusion to the corresponding syringe pump according to a sequence of infusion operations of the plurality of syringe pumps. When detecting that the infusion operation of the syringe pump in the first position is completed or about to be completed, the Dock can send the start-up instruction for the infusion to the syringe pump in the second position after the first position, so that the syringe pump in the second position can execute start-up operation for the infusion after receiving the start-up instruction for the infusion sent by the dock. The user can complete the start-up, shutdown or standby operations of the syringe pump 10 by triggering the switch key area 516. In one embodiment, the push block 1140 can be installed with a pressure sensor, so that when the push block 1140 abuts against the piston handle 606, the pressure sensor which is installed on the push block 1140 can output a feedback signal with pressure information to the processor 150, so that when the processor 150 receives the feedback signal transmitted by the pressure sensor installed on the push block 1140, it can be determined that the push block 1140 abuts against the piston handle 606. In this way, when the processor 150 receives the feedback signals outputted by the second position sensor, the third position sensor and the pressure sensor installed on the push block 1140, and receives the start-up instruction for the infusion after the user sets the infusion parameter content, the processor 150 can control the second motor speed of the drive mechanism 133 according to a preset infusion parameter content, for continuing the infusion operation.

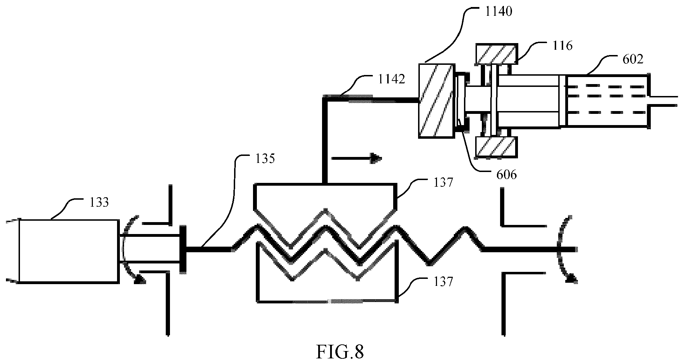

Please also refer to FIG. 8, which shows a schematic diagram when a syringe pump starts infusion operation in an embodiment of the present disclosure.

In this embodiment, the push-pull mechanism 114 is operable to clamp the piston handle 606 of the syringe 60. The processor 150 inside the syringe pump 10 sends instructions, such as speed, position and so on, to drive the power device (such as the drive mechanism 133) through a power driving circuit 130. The drive mechanism 133 drives a screw 135 and a screw nut 137 through a deceleration mechanism to convert a rotational motion of the drive mechanism 133 into a linear motion of the screw nut 137. The screw nut 137 is connected with the connection rod 1142 of the syringe pump 10. The connection rod 1142 is connected with the push block 1140. The push block 1140 can push the piston handle 660 of the matched syringe 60 for injection and infusion. Through the rotation speed of the drive mechanism 133 (such as the second motor speed), the push speed of the drive mechanism 133 to the matched syringe 60 can be adjusted, such that the given infusion amount and infusion speed can be adjusted.

In one embodiment, when the syringe pump 10 is started up or during the replacement of the syringe 60, the processor 150 can display a pipe loading guidance interface on the first display screen, which includes but is not limited to, an installation direction of the syringe 60, matters which needs attention during the installation, etc., so as to facilitate the user to accurately install the syringe 60. In other embodiments, the processor 150 may control the first display screen to display the pipe loading guidance interface when or before the installation state of the syringe 60 inside the syringe pump 10 is detected as normal.

In one embodiment, one display screen 160 is installed on the pump door 112, so that the infusion parameter setting interface 510 and the function operation interface 512 can be set in different areas of the display screen 160.

In one embodiment, after detecting the state of the pump door 112 and the installation state of the syringe 60 inside the syringe pump 10, the processor 150 can determine preset amount information of the liquid inside the syringe 10. For example, the processor 150 determines the preset amount information of the liquid inside the syringe 10 during a period from a time when detecting that the pump door 112 is opened to a time when detecting that the pump door 112 is closed. The processor 150 can detect outer diameter information of the syringe 60 and length information of the liquid inside the syringe 60 along a length direction of the piston handle 606 of the syringe 60 through the sensor, and then determine the preset amount information of the liquid inside the syringe 60 based on the outer diameter information of the syringe and the length information of the liquid. In other embodiments, the user can also set the preset amount information in the infusion parameter setting interface 510.

During the infusion operation, the liquid inside the syringe 60 gradually decreases. Further, the processor 150 can determine volume information for the injected liquid and the volume information for the remaining liquid inside the syringe 60 according to the operation of the drive mechanism 133. The processor 150 can also determine a progress information for the infusion according to the volume information for the remaining liquid and the infusion parameter content, such as the processor 150 can determine a remaining infusion time. The processor 150 also controls the display screen 160 to display the progress information for the infusion.

At present, the syringe pump adopts the serial design in the preparation process before starting up the infusion. After the startup, the syringe pump implements a self-inspection, and start to identify the state of the syringe after the self-inspection is succeeded. When the user opens the pump door to install the syringe, the push-pull mechanism is pulled out. After the syringe is successfully installed inside the syringe pump and the push-pull mechanism is successfully clamped with the piston handle, the infusion parameter setting interface will be displayed on the display screen for the user to edit or confirm. In which, it takes tens of seconds just for the push-pull mechanism to be clamped with the piston handle, so the whole process is very long, and the user can only wait, which is very unreasonable.

The above syringe pump control method can reduce the time cost of the infusion task by parallel processing the self-inspection operation of the syringe pump and the installation of syringe. In addition, when the syringe pump detects that the pump door is closed and the installation state of the syringe inside the syringe pump is normal, it controls the display screen to display the infusion parameter setting interface, which can also reduce the waste of the infusion time caused by waiting for the push-pull mechanism to run for a preset time to abut against the piston handle and then displaying the infusion parameter setting interface. The above injection pump control method can detect and control the operation of the push-pull mechanism while inputting and/or confirming the infusion parameters through the infusion parameter setting interface, which is also conducive to reducing the time cost of the infusion operation. In addition, in order to guarantee the safe use of the syringe pump, the user can start up the infusion only after the push-pull mechanism is clamped correctly with the piston handle and the infusion parameters of the syringe pump are set, which can effectively shorten the waiting time of the user and do no harm the safe use of the syringe pump.

In the above embodiments, the description of each embodiment has its own emphasis. For the parts not detailed in one embodiment, please refer to the relevant description of other embodiments.

In several embodiments provided in this disclosure, it should be understood that the disclosed devices can be realized in other ways. For example, the device embodiments described above are only schematic, for example, the division of the components is only a logical function division, and there can be another division mode in actual implementation, for example, multiple units or components can be combined or integrated into another system, or some features can be ignored or not executed. On the other hand, the mutual coupling or direct coupling or communication connection shown or discussed can be indirect coupling or communication connection through some interfaces, devices or units, and can be electrical or in other forms.

The unit described as a separate component may or may not be physically separated, and the component displayed as a unit may or may not be a physical unit, that is, it may be located in one place or distributed to multiple network units. Some or all of the units can be selected according to the actual needs to achieve the purpose of the embodiment.

In addition, each functional unit in each embodiment of this disclosure can be integrated into one processing unit. Each unit can exist separately, or two or more units can be integrated into one unit. The above integrated units can be realized in the form of hardware or software functional units.

The embodiments of this disclosure have been introduced in detail above. In this disclosure, specific examples have been operable to explain the principle and implementation mode of this disclosure. The description of the above embodiments is only operable to help those skilled in the art to understand the method and core idea of this disclosure. Meanwhile, for those skilled in the art, there will be changes in the specific implementation mode and application scope according to the idea of this disclosure. In conclusion, the contents of the specification should not be understood as restrictions on this disclosure.

## Claims

1. A syringe pump (10), wherein the syringe pump (10) is used together with a syringe (60), and the syringe pump (10) comprises a pump body (110), a drive mechanism (133), a processor (150), a display screen (160) and a pump door (112) which is rotatably installed on the pump body (110); wherein the processor (150) is operable to:
detect a state of the pump door (112) and an installation state of the syringe (60) inside the syringe pump (10); **characterized in that** the processor (150) is further operable to:
control the display screen (160) to display an infusion parameter setting interface (510), when detecting that the pump door (112) is closed and the installation state of the syringe (60) inside the syringe pump (10) is normal; wherein the infusion parameter setting interface (510) comprises an infusion parameter setting item which is operable to present one or more infusion parameter contents; and
drive the drive mechanism (133) to operate, according to at least one infusion parameter content, to move a liquid inside the syringe (60) along an infusion direction.

2. The syringe pump (10) according to claim 1, wherein the syringe pump (10) further comprises a propulsion mechanism (114); and wherein when controlling the display screen (160) to display the infusion parameter setting interface, the processor (150) is further operable to:
drive the drive mechanism (133) and control the propulsion mechanism (114) to move towards a piston handle (606) of the syringe (60).

3. The syringe pump (10) according to claim 2, wherein the piston handle (606) of the syringe (60) and the propulsion mechanism (114) are spaced by a preset distance along a length direction of the piston handle (606); and
the processor (150) is further operable to:
drive the drive mechanism (133) to operate at a first motor speed to enable the propulsion mechanism (114) to move towards the piston handle (606) of the syringe (60), wherein the propulsion mechanism (114) abuts against the piston handle (606) after the drive mechanism (133) operates at the first motor speed for a preset time;
drive the drive mechanism (133) to operate at a second motor speed according to the at least one infusion parameter content; and
control a relative movement between the piston handle (606) of the syringe (60) and a syringe body (602), to move the liquid inside the syringe (60) along the infusion direction.

4. The syringe pump (10) according to claim 1, wherein after detecting the state of the pump door (112) and the installation state of the syringe (60) inside the syringe pump (10), the processor (150) is further operable to:
detect a device state of the syringe pump (10) during a period from a time when the pump door (112) is opened to a time when the pump door (112) is closed, when detecting that the pump door (112) is opened and detecting that the pump door (112) is closed; and
after detecting that the pump door (112) is closed, control the display screen (160) to display the infusion parameter setting interface (510), when detecting that the device state of the syringe pump (10) is normal and the installation state of the syringe (60) inside the syringe pump (10) is normal.

5. The syringe pump (10) according to claim 4, wherein the processor (150) is further operable to:
implement sound self-inspection, processor self-inspection, external storage device self-inspection, alarm lamp self-inspection, power supply self-inspection and sensor self-inspection, to determine the device state of the syringe pump (10); and
in response to each of the sound self-inspection, the processor self-inspection, the external storage device self-inspection, the alarm lamp self-inspection, the power supply self-inspection and the sensor self-inspection passing, determine that the device state of the syringe pump (10) is normal.

6. The syringe pump (10) according to claim 1, wherein the processor (150) is further operable to:
detect a trigger event of a user on the infusion parameter setting interface (510) and adjust the infusion parameter content according to the trigger event; or
after detecting the state of the pump door (112) and the installation state of the syringe (60) inside the syringe pump (10), control the syringe pump (10) to implement start-up operation when detecting that the pump door (112) is opened and no syringe is installed inside the syringe pump (10); or
after detecting the state of the pump door (112) and the installation state of the syringe (60) inside the syringe pump (10), control the display screen (160) to display a pipe loading guidance interface, when or before detecting that the installation state of the syringe (60) inside the syringe pump (10) is normal.

7. The syringe pump (10) according to claim 1, wherein the syringe pump (10) further comprises a sensor which is installed at a target position; wherein after detecting the state of the pump door (112) and the installation state of the syringe (60) inside the syringe pump (10), the processor (150) is further operable to:
determine the installation state of the syringe (60) using the sensor, when detecting that the state of the pump door (112) is switched from an open state to a closed state.

8. The syringe pump (10) according to claim 7, wherein the sensor comprises a first sensor and a second sensor, the syringe pump (10) comprises a receiving groove (116) and a handle clamping mechanism (118) which is installed inside the pump body (110), the first sensor is installed inside the receiving groove (116) and the second sensor is installed on the handle clamping mechanism (118);
when the syringe (60) is installed inside the syringe pump (10); the first sensor outputs a first feedback signal and the second sensor outputs a second feedback signal, if a flange (604) on a syringe body (602) is received inside the receiving groove (116) on the pump body (110) of the syringe pump (10) and the syringe body (602) is pressed by the handle clamping mechanism (118) on the pump body (110) of the syringe pump (10);
wherein the processor (150) is further operable to determine that the installation state of the syringe (60) is normal when receiving the first feedback signal and the second feedback signal.

9. The syringe pump (10) according to claim 8, wherein the sensor comprises a third sensor, the propulsion mechanism (114) comprises a push block (1140), wherein the third sensor is installed on the push block (1140); when the propulsion mechanism (114) of the syringe pump (10) abuts against a piston handle (606) of the syringe (60), the push block (1140) contacts with the piston handle (606) and the third sensor outputs a third feedback signal; and
wherein the processor (150) is further operable to:
determine whether the first feedback signal, the second feedback signal and the third feedback signal are received; and
drive the drive mechanism (133) to operate, according to the at least one infusion parameter content, to move the liquid inside the syringe (60) along the infusion direction, when receiving the first feedback signal, the second feedback signal and the third feedback signal.

10. The syringe pump (10) according to claim 1, wherein after detecting the state of the pump door (112) and the installation state of the syringe (60) inside the syringe pump (10), the processor (150) is further operable to:
determine preset amount information of the liquid inside the syringe (60).

11. The syringe pump (10) according to claim 10, wherein the processor (150) is further operable to:
detect outer diameter information of the syringe (60) and length information of the liquid inside (60) the syringe along a length direction of a piston handle (606) of the syringe (60); and
determine the preset amount information of the liquid inside the syringe (60) based on the outer diameter information of the syringe (60) and the length information of the liquid.

12. The syringe pump (10) according to claim 11, wherein the processor (150) is further operable to:
determine volume information for a remaining liquid inside the syringe (60) according to operation of the drive mechanism (133);
determine infusion progress information according to the volume information for the remaining liquid and the infusion parameter content; and
control the display screen (160) to display the infusion progress information.

13. A syringe pump system, comprising a syringe (60) and the syringe pump (10) according to any one of claims 1 to 12.

## Patentansprüche

1. Eine Spritzenpumpe (10), wobei die Spritzenpumpe (10) zusammen mit einer Spritze (60) verwendet wird und die Spritzenpumpe (10) einen Pumpenkörper (110), einen Antriebsmechanismus (133), einen Prozessor (150), ein Display (160) und eine Pumpentür (112) umfasst, die drehbar am Pumpenkörper (110) angebracht ist; wobei der Prozessor (150) betrieblich in der Lage ist:
einen Zustand der Pumpentür (112) und einen Einbauzustand der Spritze (60) innerhalb der Spritzenpumpe (10) zu erfassen; **dadurch gekennzeichnet, dass** der Prozessor (150) ferner betrieblich in der Lage ist:
das Display (160) zur Anzeige einer Infusionsparameter-Einstellungsschnittstelle (510) zu steuern, wenn erkannt wird, dass die Pumpentür (112) geschlossen ist und der Einbauzustand der Spritze (60) innerhalb der Spritzenpumpe (10) normal ist; wobei die Infusionsparameter-Einstellungsschnittstelle (510) ein Infusionsparameter-Einstellungselement umfasst, das so betrieben werden kann, dass es einen oder mehrere Infusionsparameterinhalte darstellt; und
der Antriebsmechanismus (133) entsprechend mindestens einem Infusionsparameterinhalt angetrieben wird, um eine Flüssigkeit innerhalb der Spritze (60) entlang einer Infusionsrichtung zu bewegen.

2. Die Spritzenpumpe (10) gemäß Anspruch 1, wobei die Spritzenpumpe (10) ferner einen Antriebsmechanismus (114) umfasst; und wobei bei der Steuerung des Displays (160) zur Anzeige der Schnittstelle zur Einstellung der Infusionsparameter der Prozessor (150) ferner betrieblich in der Lage ist:
den Antriebsmechanismus (133) anzutreiben und den Antriebsmechanismus (114) so zu steuern, dass er sich in Richtung eines Kolbengriffs (606) der Spritze (60) bewegt.

3. Die Spritzenpumpe (10) nach Anspruch 2, wobei der Kolbengriff (606) der Spritze (60) und der Antriebsmechanismus (114) entlang einer Längsrichtung des Kolbengriffs (606) sich in einem voreingestellten Abstand voneinander befinden; und
der Prozessor (150) ferner betrieblich in der Lage ist:
den Antriebsmechanismus (133) so anzutreiben, dass er mit einer ersten Motordrehzahl arbeitet, damit sich der Antriebsmechanismus (114) in Richtung des Kolbengriffs (606) der Spritze (60) bewegen kann, wobei der Antriebsmechanismus (114) an den Kolbengriff (606) stößt, nachdem der Antriebsmechanismus (133) für eine voreingestellte Zeit mit der ersten Motordrehzahl gearbeitet hat;
den Antriebsmechanismus (133) so anzutreiben, dass er mit einer zweiten Motordrehzahl entsprechend dem mindestens einen Infusionsparameterinhalt arbeitet; und
eine Relativbewegung zwischen dem Kolbengriff (606) der Spritze (60) und einem Spritzenkörper (602) zu steuern, um die Flüssigkeit innerhalb der Spritze (60) entlang der Infusionsrichtung zu bewegen.

4. Die Spritzenpumpe (10) nach Anspruch 1, wobei der Prozessor (150) nach dem Erfassen des Zustands der Pumpentür (112) und des Einbauzustands der Spritze (60) innerhalb der Spritzenpumpe (10) ferner betrieblich in der Lage ist:
einen Gerätestatus der Spritzenpumpe (10) während eines Zeitraums vom Öffnen der Pumpentür (112) bis zum Schließen der Pumpentür (112) zu erkennen, wenn das Öffnen der Pumpentür (112) und das Schließen der Pumpentür (112) erfasst werden; und
nach dem Erkennen, dass die Pumpentür (112) geschlossen ist, das Display (160) so zu steuern, dass die Infusionsparameter-Einstellungsschnittstelle (510) angezeigt wird, wenn erkannt wird, dass der Gerätestatus der Spritzenpumpe (10) normal ist und der Einbauzustand der Spritze (60) innerhalb der Spritzenpumpe (10) normal ist.

5. Die Spritzenpumpe (10) nach Anspruch 4, wobei der Prozessor (150) ferner betrieblich in der Lage ist:
eine eingehende Selbstprüfung, eine Selbstprüfung des Prozessors, eine Selbstprüfung des externen Speichergeräts, eine Selbstprüfung der Alarmleuchte, eine Selbstprüfung der Stromversorgung und eine Selbstprüfung des Sensors durchzuführen, um den Gerätestatus der Spritzenpumpe (10) zu bestimmen; und
als Reaktion auf das Bestehen jeder der eingehenden Selbstprüfung, der Selbstprüfung des Prozessors, der Selbstprüfung des externen Speichergeräts, der Selbstprüfung der Alarmleuchte, der Selbstprüfung der Stromversorgung und der Selbstprüfung des Sensors zu bestimmen, dass der Gerätestatus der Spritzenpumpe (10) normal ist.

6. Die Spritzenpumpe (10) nach Anspruch 1, wobei der Prozessor (150) ferner betrieblich in der Lage ist:
ein Auslöseereignis eines Benutzers über die Schnittstelle zur Einstellung der Infusionsparameter (510) zu erfassen und die Infusionsparameter entsprechend dem Auslöseereignis anzupassen; oder
nach dem Erkennen des Zustands der Pumpentür (112) und des Einbauzustands der Spritze (60) innerhalb der Spritzenpumpe (10) die Spritzenpumpe (10) so steuern, dass sie einen Startvorgang ausführt, wenn erkannt wird, dass die Pumpentür (112) geöffnet ist und keine Spritze innerhalb der Spritzenpumpe (10) installiert ist; oder
nach dem Erfassen des Zustands der Pumpentür (112) und des Einbauzustands der Spritze (60) innerhalb der Spritzenpumpe (10) das Display (160) so steuern, dass eine Schnittstelle zur Anleitung zum Füllen der Leitung angezeigt wird, wenn oder bevor erfasst wird, dass der Einbauzustand der Spritze (60) innerhalb der Spritzenpumpe (10) normal ist.

7. Die Spritzenpumpe (10) nach Anspruch 1, wobei die Spritzenpumpe (10) ferner einen Sensor umfasst, der an einer Zielposition installiert ist; wobei nach dem Erfassen des Zustands der Pumpentür (112) und des Einbauzustands der Spritze (60) innerhalb der Spritzenpumpe (10) der Prozessor (150) ferner betrieblich in der Lage ist:
den Installationszustand der Spritze (60) unter Verwendung des Sensors zu bestimmen, wenn festgestellt wird, dass der Zustand der Pumpentür (112) von einem offenen Zustand in einen geschlossenen Zustand gewechselt ist.

8. Die Spritzenpumpe (10) nach Anspruch 7, wobei der Sensor einen ersten Sensor und einen zweiten Sensor umfasst, die Spritzenpumpe (10) eine Aufnahmenut (116) und einen Griffklemmmechanismus (118) umfasst, der innerhalb des Pumpenkörpers (110) angebracht ist, der erste Sensor innerhalb der Aufnahmenut (116) installiert ist und der zweite Sensor auf dem Griffklemmmechanismus (118) installiert ist;
bei in der Spritzenpumpe (10) installierter Spritze (60) gibt der erste Sensor ein erstes Rückmeldesignal aus und der zweite Sensor ein zweites Rückmeldesignal, wenn ein Flansch (604) an einem Spritzenkörper (602) in der Aufnahmenut (116) am Pumpenkörper (110) der Spritzenpumpe (10) aufgenommen wird und der Spritzenkörper (602) durch den Griffklemmmechanismus (118) an den Pumpenkörper (110) der Spritzenpumpe (10) gedrückt wird;
wobei der Prozessor (150) ferner betrieblich in der Lage ist, dass er beim Empfang des ersten Rückmeldesignals und des zweiten Rückmeldesignals feststellt, dass der Einbauzustand der Spritze (60) normal ist.

9. Die Spritzenpumpe (10) nach Anspruch 8, wobei der Sensor einen dritten Sensor umfasst, der Antriebsmechanismus (114) einen Druckblock (1140) umfasst und wobei der dritte Sensor auf dem Druckblock (1140) installiert ist; wenn der Antriebsmechanismus (114) der Spritzenpumpe (10) an einem Kolbengriff (606) der Spritze (60) anliegt, der Druckblock (1140) mit dem Kolbengriff (606) in Kontakt kommt und der dritte Sensor ein drittes Rückmeldesignal ausgibt; und
wobei der Prozessor (150) ferner betrieblich in der Lage ist:
zu bestimmen, ob das erste Rückmeldesignal, das zweite Rückmeldesignal und das dritte Rückmeldesignal empfangen werden; und
den Antriebsmechanismus (133) entsprechend dem mindestens einen Infusionsparameterinhalt zu betreiben, um die Flüssigkeit innerhalb der Spritze (60) entlang der Infusionsrichtung zu bewegen, wenn das erste Rückmeldesignal, das zweite Rückmeldesignal und das dritte Rückmeldesignal empfangen werden.

10. Die Spritzenpumpe (10) nach Anspruch 1, wobei der Prozessor (150) nach dem Erfassen des Zustands der Pumpentür (112) und des Einbauzustands der Spritze (60) in der Spritzenpumpe (10) ferner betrieblich in der Lage ist:
die voreingestellte Mengeninformation der Flüssigkeit innerhalb der Spritze (60) zu bestimmen.

11. Die Spritzenpumpe (10) nach Anspruch 10, wobei der Prozessor (150) ferner betrieblich in der Lage ist:
Informationen über den Außendurchmesser der Spritze (60) zu erfassen sowie Informationen über die Länge der Flüssigkeit innerhalb (60) der Spritze entlang einer Längsrichtung eines Kolbengriffs (606) der Spritze (60); und
die voreingestellte Mengeninformation der Flüssigkeit in der Spritze (60) auf der Grundlage der Außendurchmesserinformation der Spritze (60) und der Längeninformation der Flüssigkeit zu bestimmen.

12. Die Spritzenpumpe (10) nach Anspruch 11, wobei der Prozessor (150) ferner betrieblich in der Lage ist:
Volumeninformationen für eine in der Spritze (60) verbleibende Flüssigkeit gemäß dem Betrieb des Antriebsmechanismus (133) zu bestimmen;
Informationen über den Infusionsfortschritt gemäß den Volumeninformationen für die verbleibende Flüssigkeit und den Infusionsparameterinhalt zu bestimmen; und
das Display (160) zur Anzeige der Informationen zum Infusionsfortschritt zu steuern.

13. Ein Spritzenpumpensystem, bestehend aus einer Spritze (60) und der Spritzenpumpe (10) nach einem der Ansprüche 1 bis 12.

## Revendications

1. Pompe de seringue (10), où la pompe de seringue (10) est utilisée conjointement avec une seringue (60), et la pompe de seringue (10) comprend un corps de pompe (110), un mécanisme d'entraînement (133), un processeur (150), un écran d'affichage (160) et une porte de pompe (112) laquelle est installée avec possibilité de rotation sur le corps de pompe (110) ; où le processeur (150) est utilisable pour :
détecter un état de la porte de pompe (112) et un état d'installation de la seringue (60) à l'intérieur de la pompe de seringue (10) ; **caractérisée en ce que** le processeur (150) est en outre utilisable pour :
commander à l'écran d'affichage (160) d'afficher une interface de réglage de paramètre de perfusion (510), lorsqu'il est détecté que la porte de pompe (112) est fermée et que l'état d'installation de la seringue (60) à l'intérieur de la pompe de seringue (10) est normal ; où l'interface de réglage de paramètre de perfusion (510) comprend un élément de réglage de paramètre de perfusion lequel est utilisable pour présenter un ou plusieurs contenus de paramètre de perfusion ; et
entraîner le mécanisme d'entraînement (133) pour le faire fonctionner, selon au moins un contenu de paramètre de perfusion, afin de déplacer un liquide à l'intérieur de la seringue (60) le long d'une direction de perfusion.

2. Pompe de seringue (10) selon la revendication 1, où la pompe de seringue (10) comprend en outre un mécanisme de propulsion (114) ; et où lorsqu'il est commandé à l'écran d'affichage (160) d'afficher l'interface de réglage de paramètre de perfusion, le processeur (150) est en outre utilisable pour :
entraîner le mécanisme d'entraînement (133) et commander au mécanisme de propulsion (114) de se déplacer vers une poignée de piston (606) de la seringue (60).

3. Pompe de seringue (10) selon la revendication 2, où la poignée de piston (606) de la seringue (60) et le mécanisme de propulsion (114) sont espacés d'une distance prédéfinie le long d'une direction de longueur de la poignée de piston (606) ; et
le processeur (150) est en outre utilisable pour :
entraîner le mécanisme d'entraînement (133) pour le faire fonctionner à une première vitesse de moteur pour permettre au mécanisme de propulsion (114) de se déplacer vers la poignée de piston (606) de la seringue (60), où le mécanisme de propulsion (114) bute contre la poignée de piston (606) après que le mécanisme d'entraînement (133) a fonctionné à la première vitesse de moteur pendant une durée prédéfinie ;
entraîner le mécanisme d'entraînement (133) pour le faire fonctionner à une seconde vitesse de moteur selon le au moins un contenu de paramètre de perfusion ; et
commander un déplacement relatif entre la poignée de piston (606) de la seringue (60) et un corps de seringue (602), pour déplacer le liquide à l'intérieur de la seringue (60) le long de la direction de perfusion.

4. Pompe de seringue (10) selon la revendication 1, où après avoir détecté l'état de la porte de pompe (112) et l'état d'installation de la seringue (60) à l'intérieur de la pompe de seringue (10), le processeur (150) est en outre utilisable pour :
détecter un état de dispositif de la pompe de seringue (10) durant une période entre un instant où la porte de pompe (112) est ouverte et un instant où la porte de pompe (112) est fermée, lorsqu'il est détecté que la porte de pompe (112) est ouverte et qu'il est détecté que la porte de pompe (112) est fermée ; et
après avoir détecté que la porte de pompe (112) est fermée, commander à l'écran d'affichage (160) d'afficher l'interface de réglage de paramètre de perfusion (510), lorsqu'il est détecté que l'état de dispositif de la pompe de seringue (10) est normal et que l'état d'installation de la seringue (60) à l'intérieur de la pompe de seringue (10) est normal.

5. Pompe de seringue (10) selon la revendication 4, où le processeur (150) est en outre utilisable pour :
mettre en œuvre une auto-inspection sonore, une auto-inspection de processeur, un auto-inspection de dispositif de stockage externe, une auto-inspection de voyant d'alarme, une auto-inspection d'alimentation électrique et une auto-inspection de capteur, afin de déterminer l'état de dispositif de la pompe de seringue (10) ; et
en réponse au fait que chacune des auto-inspection sonore, auto-inspection de processeur, auto-inspection de dispositif de stockage externe, auto-inspection de voyant d'alarme, auto-inspection d'alimentation électrique et auto-inspection de capteur est concluante, déterminer que l'état de dispositif de la pompe de seringue (10) est normal.

6. Pompe de seringue (10) selon la revendication 1, où le processeur (150) est en outre utilisable pour :
détecter un événement de déclenchement d'un utilisateur sur l'interface de réglage de paramètre de perfusion (510) et ajuster le contenu du paramètre de perfusion selon l'événement de déclenchement ; ou
après avoir détecté l'état de la porte de pompe (112) et l'état d'installation de la seringue (60) à l'intérieur de la pompe de seringue (10), commander à la pompe de seringue (10) de réaliser une opération de démarrage lorsqu'il est détecté que la porte de pompe (112) est ouverte et qu'aucune seringue n'est installée à l'intérieur de la pompe de seringue (10) ; ou
après avoir détecté l'état de la porte de pompe (112) et l'état d'installation de la seringue (60) à l'intérieur de la pompe de seringue (10), commander à l'écran d'affichage (160) d'afficher une interface de guide de charge de tuyau, lorsqu'il est détectée ou avant qu'il soit détecté que l'état d'installation de la seringue (60) à l'intérieur de la pompe de seringue (10) est normal.

7. Pompe de seringue (10) selon la revendication 1, où la pompe de seringue (10) comprend en outre un capteur lequel est installé à une position cible ; où après avoir détecté l'état de la porte de pompe (112) et l'état d'installation de la seringue (60) à l'intérieur de la pompe de seringue (10), le processeur (150) est en outre utilisable pour :
déterminer l'état d'installation de la seringue (60) à l'aide du capteur, lorsqu'il est détecté que l'état de la porte de pompe (112) est passé d'un état ouvert à un état fermé.

8. Pompe de seringue (10) selon la revendication 7, où le capteur comprend un premier capteur et un deuxième capteur, la pompe de seringue (10) comprend une rainure de réception (116) et un mécanisme de fixation de poignée (118) lequel est installé à l'intérieur du corps de pompe (110), le premier capteur est installé à l'intérieur de la rainure de réception (116) et le deuxième capteur est installé sur le mécanisme de fixation de poignée (118) ;
lorsque la seringue (60) est installée à l'intérieur de la pompe de seringue (10) ; le premier capteur fournit un premier signal de retour d'information et le deuxième capteur fournit un deuxième signal de retour d'information, si une ailette (604) sur un corps de seringue (602) est reçue à l'intérieur de la rainure de réception (116) sur le corps de pompe (110) de la pompe de seringue (10) et que le corps de seringue (602) est poussé par le mécanisme de fixation de poignée (118) sur le corps de pompe (110) de la pompe de seringue (10) ;
où le processeur (150) est en outre utilisable pour déterminer que l'état d'installation de la seringue (60) est normal à la réception du premier signal de retour d'information et du deuxième signal de retour d'information.

9. Pompe de seringue (10) selon la revendication 8, où le capteur comprend un troisième capteur, le mécanisme de propulsion (114) comprend un bloc de poussée (1140), où le troisième capteur est installé sur le bloc de poussée (1140) ; lorsque le mécanisme de propulsion (114) de la pompe de seringue (10) bute contre une poignée de piston (606) de la seringue (60), le bloc de poussée (1140) est en contact avec la poignée de piston (606) et le troisième capteur fournit un troisième signal de retour d'information ; et
où le processeur (150) est en outre utilisable pour :
déterminer si le premier signal de retour d'information, le deuxième signal de retour d'information et le troisième signal de retour d'information sont reçus ; et
entraîner le mécanisme d'entraînement (133) pour le faire fonctionner, selon le au moins un contenu de paramètre de perfusion, afin de déplacer un liquide à l'intérieur de la seringue (60) le long de la direction de perfusion, à la réception du premier signal de retour d'information, du deuxième signal de retour d'information et du troisième signal de retour d'information.

10. Pompe de seringue (10) selon la revendication 1, où après avoir détecté l'état de la porte de pompe (112) et l'état d'installation de la seringue (60) à l'intérieur de la pompe de seringue (10), le processeur (150) est en outre utilisable pour :
déterminer une information de quantité prédéfinie du liquide à l'intérieur de la seringue (60).

11. Pompe de seringue (10) selon la revendication 10, où le processeur (150) est en outre utilisable pour :
détecter une information de diamètre extérieur de la seringue (60) et une information de longueur du liquide à l'intérieur de la seringue (60) le long d'une direction de longueur d'une poignée de piston (606) de la seringue (60) ; et
déterminer l'information de quantité prédéfinie du liquide à l'intérieur de la seringue (60) sur la base de l'information de diamètre extérieur de la seringue (60) et de l'information de longueur du liquide.

12. Pompe de seringue (10) selon la revendication 11, où le processeur (150) est en outre utilisable pour :
déterminer une information de volume pour un liquide restant à l'intérieur de la seringue (60) selon une opération du mécanisme d'entraînement (133) ;
déterminer une information d'avancement de perfusion selon l'information de volume pour le liquide restant et le contenu de paramètre de perfusion ; et
commander à l'écran d'affichage (160) d'afficher l'information d'avancement de perfusion.

13. Système de pompe de seringue, comprenant une seringue (60) et la pompe de seringue (10) selon l'une quelconque des revendications 1 à 12.
